(19)

Europäisches
Patentamt
European
Patent Office
Office européen
des brevets

(11) **EP 2 354 991 A1**

(12) **EUROPEAN PATENT APPLICATION**

(43) Date of publication:
**10.08.2011 Bulletin 2011/32**

(51) Int Cl.:
***G06F 19/00*** (2011.01)

(21) Application number: **10152575.6**

(22) Date of filing: **03.02.2010**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA RS** | (72) Inventors:<br>• **Alferness, Clifton A.**<br>  **Port Orchard, WA 98367 (US)**<br>• **Bardy, Gust H.**<br>  **Redmond, WA 98053 (US)** |
| (71) Applicants:<br>• **Alferness, Clifton A.**<br>  **Port Orchard, WA 98367 (US)**<br>• **Bardy, Gust H.**<br>  **Redmond, WA 98053 (US)** | (74) Representative: **Curley, Donnacha John et al**<br>  **Hanna Moore & Curley**<br>  **13 Lower Lad Lane**<br>  **Dublin 2 (IE)** |

(54) **System and method for actively managing type 2 diabetes mellitus on a personalized basis**

(57)     A system (180) and method for actively managing Type 2 diabetes mellitus on a personalized basis is provided. A model of glycemic effect for a Type 2 diabetic patient (31) for digestive response (61) is established. The digestive response model (61) is adjusted for a degree of insulin resistance (194) experienced by the patient (31). A rise in postprandial blood glucose (121) through food ingestion of a planned meal (102) is estimated in proportion to the adjusted digestive response model (61). The tool also allows for the avoidance of hypoglycemic episodes by medications.

# Fig. 15.

**Description**

<u>Field</u>

This application relates in general to Type 2 diabetes mellitus management and, in particular, to a system and method for actively managing Type 2 diabetes mellitus on a personalized basis.

<u>Background</u>

Diabetes mellitus, or simply, "diabetes," is an incurable chronic disease. Type 1 diabetes is caused by the destruction of pancreatic beta cells in the Islets of Langerhans through autoimmune attack. Type 2 diabetes is due to defective insulin secretion, insulin resistance, or reduced insulin sensitivity. Gestational diabetes first appears during pregnancy and generally resolves after childbirth, absent preexisting weak pancreatic function. Less common forms of diabetes include thiazide-induced diabetes, and diabetes caused by chronic pancreatitis, tumors, hemochromatosis, steroids, Cushing's disease, and acromegaly.

Type 2 diabetes is a progressive disease with increasing risks and complications due to increased insulin resistance and diminished insulin secretion. Type 2 diabetics generally present less labile metabolic profiles, but face more chronic conditions than Type 1 diabetics. These conditions include cardiovascular disease, retinopathy, neuropathy, nephropathy, and non-alcoholic steatohepatitis.

Type 2 diabetes management adapts progressively with disease stage. Initially, Type 2 diabetes is managed through changes in physical activity, diet, and weight, which may temporarily restore normal insulin sensitivity. As insulin production or uptake become impaired, antidiabetic medications may become necessary to increase insulin production, decrease insulin resistance, and help regulate inappropriate hepatic glucose release. Insulin therapy is generally started after insulin production ceases.

Effective diabetes management requires effort. Inexperience, lack of self discipline, and indifference can result in poor diabetes management. Intuition is not infallible and well-intentioned insulin dosing is of little use if the patient forgets to actually take his insulin or disregards dietary planning. Similarly, a deviation from dietary planning followed by a remedial insulin dosage can result in undesirable and often dangerous blood glucose oscillations. Physiological factors, well beyond the value of intuition or skill, such as illness, stress, and general well-being, can also complicate management, particularly during end-stage Type 2 diabetes.

Despite the importance of effective management, Type 2 diabetics seldom receive direct day-to-day oversight. Physician experience, patient rapport, and constrained clinic time pose limits on the amount and quality of oversight provided. Physicians are often removed in time and circumstance from significant metabolic events and blood glucose aberrations, often significant, may not present in-clinic when a physician can actually observe them. Primary care and especially endocrinologist visits occur infrequently and at best provide only a "snapshot" of diabetes control. For instance, glycated hemoglobin (HbA1c) is tested every three to six months to evaluate long-term control, yet reflects a bias over more recent blood glucose levels and has no bearing on brief very high or very low blood glucose levels that can carry serious adverse consequences.

These above delineated limitations in care notwithstanding, existing approaches to diabetes management still rely on physician decision-making. For instance, U.S. Patent No. 6,168,563, to Brown, discloses a healthcare maintenance system based on a hand-held device. Healthcare data, such as blood glucose, can be uploaded onto a program cartridge for healthcare professional analysis at a centralized location. Healthcare data can also be directly obtained from external monitoring devices, including blood glucose monitors. At the centralized location, blood glucose test results can be matched with quantitative information on medication, meals, or other factors, such as exercise. Changes in medication dosage or modification to the patient's s monitoring schedule can be electronically sent back to the patient. However, decision making on day-to-day (and even hour-to-hour) diabetes management through interpretation of uploaded healthcare data remains an offline process, being discretionary to the remote healthcare professional and within his sole control and timing.

Similarly, U.S. Patent No. 6,024,699, to Surwit et al. ("Surwit"), discloses monitoring, diagnosing, prioritizing, and treating medical conditions of a plurality of remotely located patients. Each patient uses a patient monitoring system that includes medicine dosage algorithms, which use stored patient data to generate dosage recommendations for the patient. A physician can modify the medicine dosage algorithms, medicine doses, and fixed or contingent self-monitoring schedules, including blood glucose monitoring through a central data processing system. Diabetes patients can upload their data to the central data processing system, which will detect any trends or problems. If a problem is detected, a revised insulin dosing algorithm, insulin dosage, or self-monitoring schedule can be downloaded to their patient monitoring system. However, any changes to diabetes management remain within the sole discretion and timing of a physician, who acts remotely in place and time via the central data processing system.

Therefore, there is a need for a progressive approach to Type 2 diabetes management with provisions for customizing glycemic control parameters to meet a diabetic's personal sensitivities and day-to-day needs without the delay inherent in current diabetes management.

## Summary

A system and method for interactively managing Type 2 diabetes on an individualized and patient-specific basis is provided for use at any time and in any place and for any diet under any metabolic circumstance. Models of glycemic effect by meals, by antidiabetic and oral medications, and by insulin are formed, as applicable, based on sensitivities particular to a diabetic patient. A rise in blood glucose is estimated based on food selections indicated by the patient, which is adjusted to compensate for the patient's specific carbohydrate sensitivity, as well as for any supervening physiological or pathophysiological influences. Similarly, the effect of any antidiabetic and oral medications is evaluated in relation to blood glucose with the goal of not only preventing hyperglycemia, but hypoglycemic episodes that interfere with day-to-day safe conduct of activities of daily living. For end-stage Type 2 diabetics, an amount of insulin necessary to counteract the rise in blood glucose over the expected time course of a meal is determined, also adjusted to match the patient's insulin sensitivity and to prevent the equally serious declines in blood glucose.

One embodiment provides a system and method for actively managing Type 2 diabetes mellitus on a personalized basis. A model of glycemic effect for a Type 2 diabetic patient for digestive response is established. The digestive response model is adjusted for the degree of insulin resistance experienced by the patient. A rise in postprandial blood glucose through food ingestion of a planned meal is estimated in proportion to the adjusted digestive response model.

The personal predictive management tool provides Type 2 diabetics with a new-found sense of personal freedom and safety by integrating the vagaries of daily blood glucose control into a holistic representation that can be applied and refined to keep pace with the unpredictable nature of daily life. The approach described herein closely approximates what a normal pancreas does by interactively guiding the individual diabetic under consideration and, over time, learning how the patient can be understood and advised.

This invention also extends beyond the prevention of hyperglycemia and includes the prevention of hypoglycemia. Hypoglycemic episodes are a bane to insulin users and can result in confusion, syncope, seizures, falls, automobile accidents, and embarrassment, all of which result from the confusing mental state that results when blood glucose falls below 65 or thereabouts in most people. As a matter of practical day-to-day diabetes management, hypoglycemia is more of a concern to the insulin user than the long term consequences of hyperglycemia. The negative consequences of hyperglycemia seem remote to most patients who fear the immediate negative consequence of hypoglycemia in any of the traditional approaches to strictly control their blood glucose. Thus, the concern over hypoglycemic symptoms often prevents patients from optimally controlling their blood glucose levels. The approach provided herein takes into account the problem of hypoglycemia with the same rigor as that applied to hyperglycemia.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

## Brief Description of the Drawings

FIGURES 1A-C are functional block diagrams showing, by way of example, a prior art diabetes management cycle for a typical Type 2 diabetic.

FIGURES 2A-C are functional block diagrams showing, by way of example, an automated diabetes management cycle for a typical Type 2 diabetic, in accordance with one embodiment.

FIGURE 3 is a process flow diagram showing personalized Type 2 diabetes mellitus modeling.

FIGURE 4 is a diagram showing a method for progressively managing the stages of Type 2 diabetes mellitus.

FIGURE 5 is a process flow diagram showing management of the early stage of Type 2 diabetes mellitus for use with the method of FIGURE 4.

FIGURE 6 is a graph showing, by way of example, a digestive response curve for a standardized test meal.

FIGURE 7 is a process flow diagram showing management of the middle stage of Type 2 diabetes mellitus for use with the method of FIGURE 4.

FIGURE 8 is a process flow diagram showing management of the end-stage of Type 2 diabetes mellitus for use with the method of FIGURE 4.

FIGURE 9 is a graph showing, by way of example, an insulin activity curve for lispro, an insulin analog.

FIGURE 10 is a diagram showing, by way of example, a screen shot of a graphical user interface for performing automated management of Type 2 diabetes, in accordance with one embodiment.

FIGURE 11 is a diagram showing, by way of example, a screen shot of a graphical user interface for selecting food combinations for use in the graphical user interface of FIGURE 10.

FIGURES 12A-C are graphs showing, by way of example, constituent and cumulative digestive response curves for a hypothetical meal.

FIGURE 13 is a diagram showing, by way of example, a screen shot of a graphical user interface for specifying insulin preparation type for use in the graphical user interface of FIGURE 10.

FIGURE 14 is a diagram showing, by way of example, a screen shot of a graphical user interface for specifying other medications for use in the graphical user interface of FIGURE 10.

FIGURE 15 is a process flow diagram showing a method for actively managing Type 2 diabetes mellitus on a personalized basis, in accordance with one embodiment.

FIGURE 16 is a process flow diagram showing a routine for refining a food data library for use with the method of FIGURE 15.

FIGURE 17 is a process flow diagram showing a routine for interacting with a patient for use with the method of FIGURE 15.

FIGURE 18 is a block diagram showing for a system for actively managing Type 2 diabetes mellitus on a personalized basis, in accordance with one embodiment.

## Detailed Description

Diabetes Management Cycles

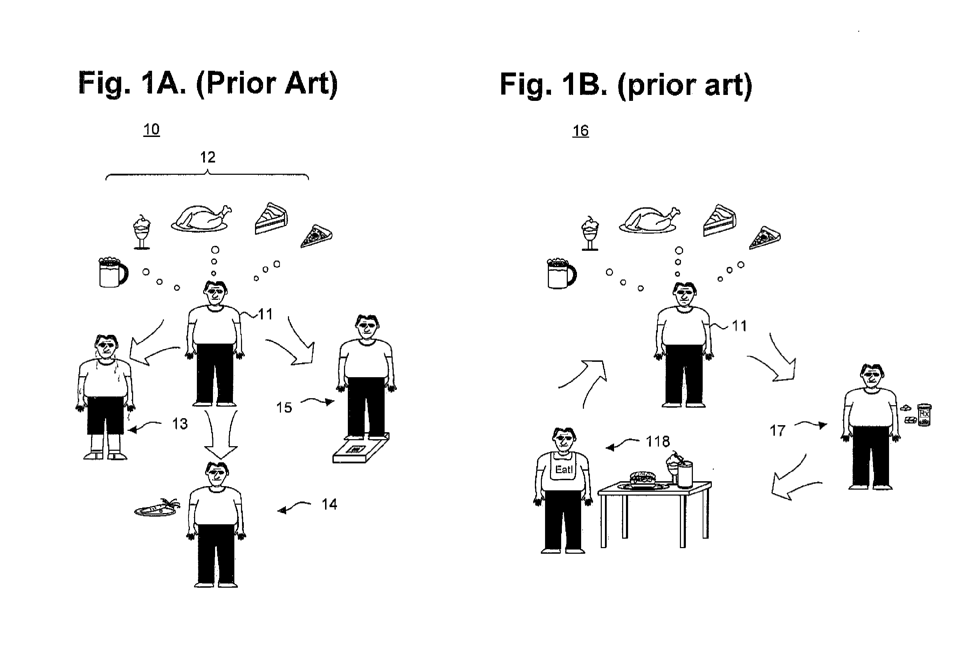

Type 2 diabetes is due to defective insulin secretion, insulin resistance, or reduced insulin sensitivity. No known preventative measures exist, but the disease has been strongly correlated to obesity and genetic predisposition. Type 2 diabetes management is performed continually on a daily basis, although the nature and degree of management intensifies as the disease progresses. FIGURES 1A-C are functional block diagrams showing, by way of example, prior art diabetes management cycles 10, 16, 19 for a Type 2 diabetic. Early stage of Type 2 diabetes can generally be controlled through lifestyle changes alone, to which antidiabetic medications and ultimately insulin therapy are eventually added.

Early stage Type 2 diabetes management focuses on lifestyle adjustments with an emphasis on basic glycemic control. Referring first to FIGURE 1A, a typical Type 2 diabetes patient 11 is often obese, although obesity is but one indicator of Type 2 diabetes, which also includes genetic predisposition and mutation of amylin genes. Diet 12 or, more bluntly, unhealthy diet, often plays a significant role. As a result, the patient 11 is urged to exercise regularly (step 13) through a combination of aerobic and resistance training, for instance, by taking a brisk 45-minute walk several times a week. In addition, the patient 11 is educated on following a healthy diet (step 14). The combination of exercise and healthy diet help to control weight (step 15), which is crucial to early stage Type 2 diabetes, as the level of insulin resistance proportionately grows with increase in body fat, particularly metabolically active visceral fat.

Effective early stage Type 2 diabetes control can temporarily restore normal insulin sensitivity, although the predisposition for insulin resistance generally remains dormant. Middle stage Type 2 diabetes eventually follows and is characterized by increasing insulin resistance and decreasing insulin production. Referring next to FIGURE 1B, antidiabetic and oral medications are generally prescribed (step 17) during the middle stage, as insulin production becomes impaired, yet partial pancreatic function remains. Lifestyle changes (step 18) in exercise, diet, and weight control continue as during the early stage. Type 2 diabetes management strives to achieve an HbA1c of 6.0-7.0.

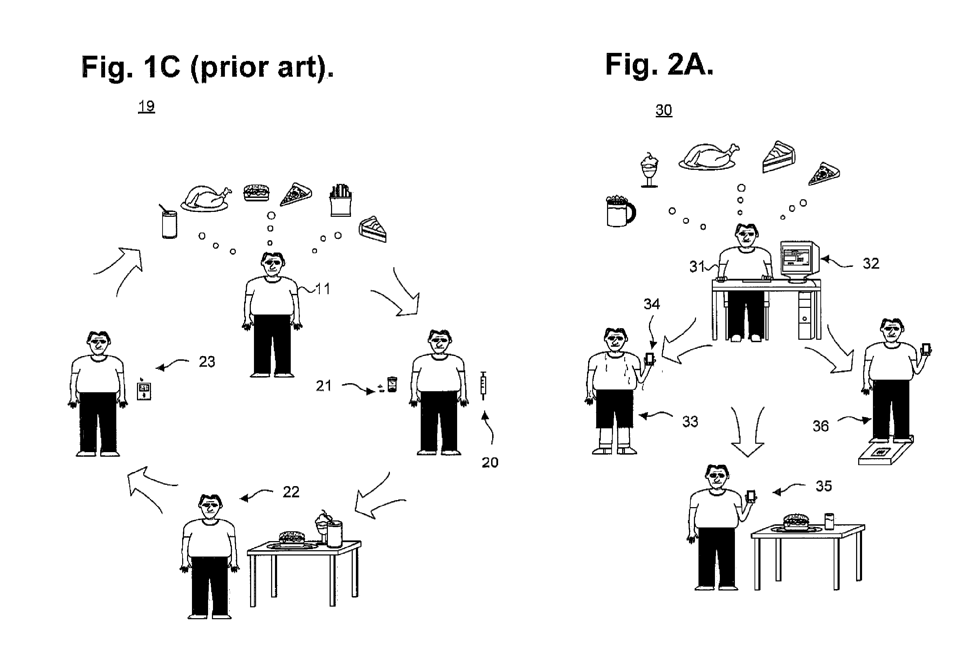

In the end-stage, pancreatic function has ceased, which necessitates commencement of insulin therapy. Referring to FIGURE 1C, insulin therapy includes both conscious meal and insulin dosage planning, as the body no longer has the innate ability to counteract blood glucose rise through naturally produced insulin. Ideally, a Type 2 diabetic's average blood glucose should be in the range of 80-120 milligrams per deciliter (mg/dL), although a range of 140-150 mg/dL is often used to prevent potentially life-threatening hypoglycemic events. When properly dosed (step 20), the insulin will return blood glucose to a normal range within two to four hours of consuming a meal, although the patient must determine the proper amount of insulin needed ahead of time based on what he plans to eat. Antidiabetic and oral medications (step 21) may also be taken, along with continued adherence to lifestyle changes (step 22). As well, beginning with insulin therapy, the patient 11 is now encouraged to regularly self-test his blood glucose (step 23).

Non-pharmacological management of Type 2 diabetes, that is, lifestyle modification, relies on the patient's personal willpower and discipline, both of which vary greatly by patient and circumstance and frequently fall short of what is necessary to improve blood glucose control. Thus, to provide increased consistency and patient awareness, Type 2 diabetes management can be automated and thereby provide each diabetic patient with better chances of effective glycemic control throughout each stage of the disease. FIGURES 2A-C are functional block diagrams showing, by way of example, automated diabetes management cycles 30, 37, 41 for a Type 2 diabetic, in accordance with one embodiment. Automation is introduced to increase the accuracy and timeliness of blood glucose control and logging, and to minimize or eliminate missteps performed by a patient resulting from pure intuition or happenstance.

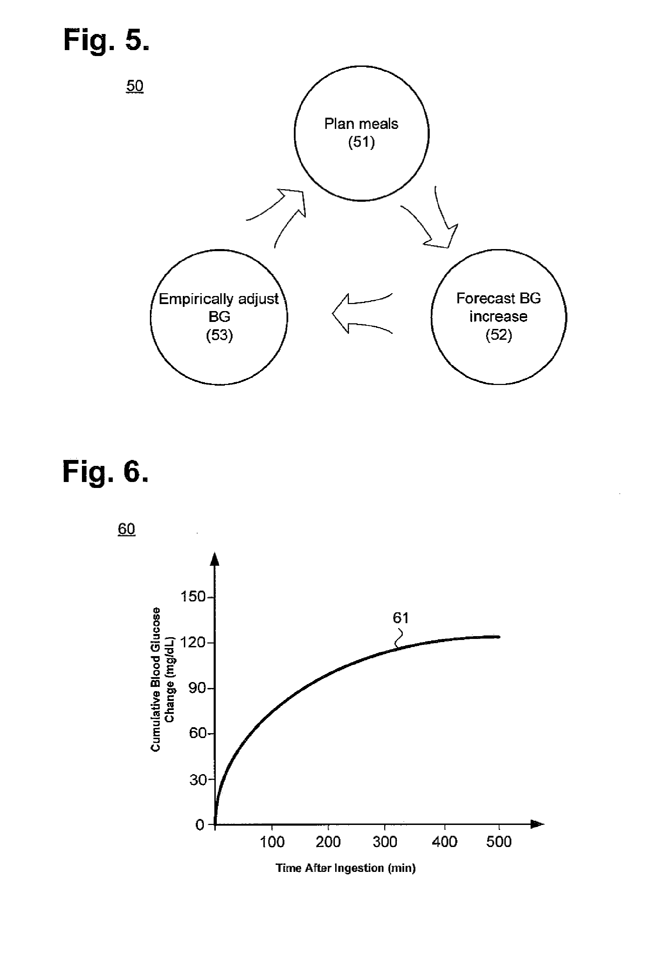

Type 2 diabetes is a progressively debilitating disorder and quality of life can best be preserved by seeding the patient's consciousness with better diabetes awareness from the earliest stages of the disease. Referring first to FIGURE 2A, a Type 2 diabetic patient 31 faces making changes to his lifestyle through exercise and physical activity (step 33), healthy diet (step 35), and weight management (step 36). Hopefully, the changes are permanent, but a diabetic 51 may lose sight of their importance, through indifference, inability to comply with the complexity of a serious change in lifestyle, or by temporary restoration of insulin sensitivity. Consequently, during early stage Type 2 diabetes, an automated diabetes management tool can be used to assist the patient 31 in planning his diet, exercise, and physical activities (step 32), and in tracking his progress (step 34) for subsequent review and analysis, as further described below with reference to FIGURE 5. The management tool helps the patient to adhere to the changes.

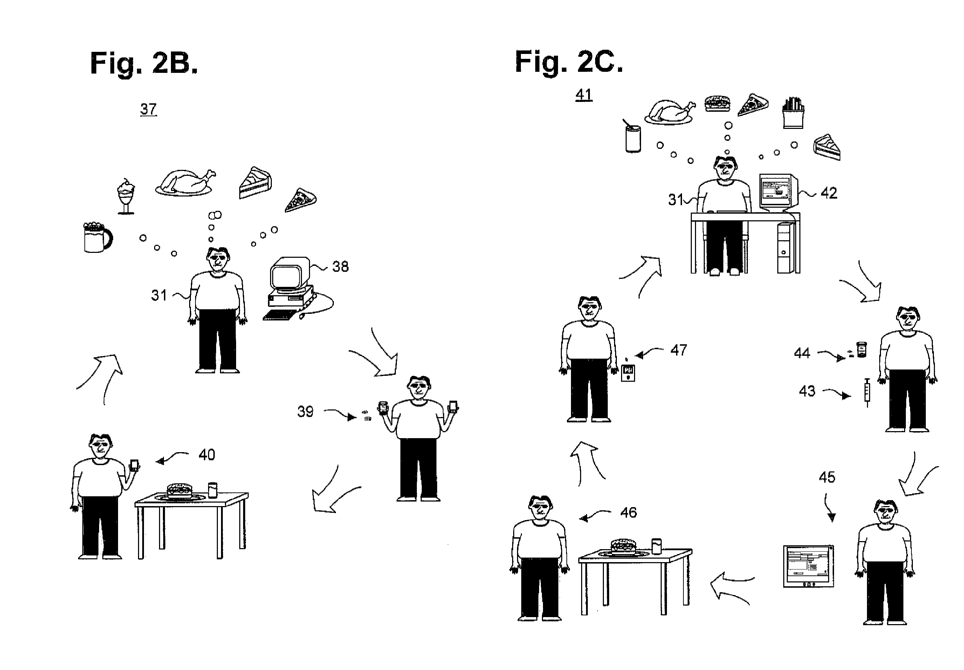

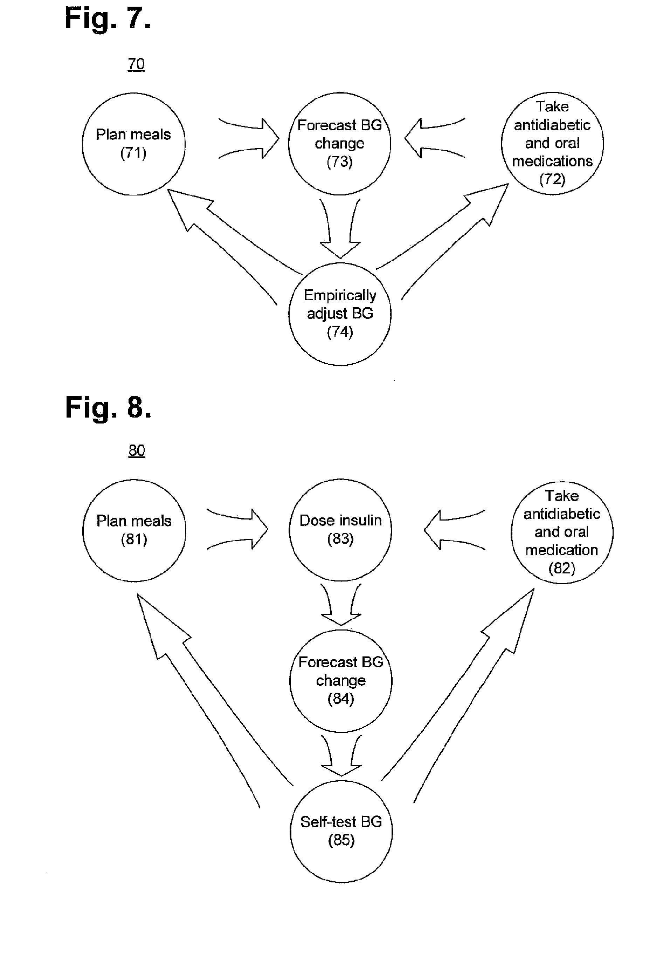

As insulin resistance increases and pancreatic function decreases, antidiabetic and oral medications generally become necessary. Referring next to FIGURE 2B, the types and timing of medications required depend upon the patient's physiology and physical tolerance. Moreover, medication may be necessary at different times of the day and in different combinations. In addition to the dietary and physical activity planning (step 38) and compliance tracking (step 40) functions of this management tool, the tool can also provide antidiabetic and oral medication dosing instructions and reminders (step 39) to the patient regarding needed actions in the near future 31, as further described below with reference to FIGURE 7.

End-stage Type 2 diabetes introduces insulin therapy. Insulin can only be dosed through cutaneous injection and must be timed against anticipated metabolism. Referring finally to FIGURE 2C, insulin requires conscious planning (step 42) and conscientious dosing (step 43), both in appropriate amount and at the correct time with respect to meals and activities to effectively lower the blood sugar and to prevent the negative consequences of hypoglycemia. The management tool applies meal and physical activity planning (step 42) and compliance and self-testing tracking (step 46) functions similar in form to the methodology of Type 1 diabetes, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference, but further includes administration of antidiabetic and oral medications (step 44), where applicable. In addition, the management tool can provide dynamic blood glucose prediction (step 45) and blood glucose self-testing integration (step 47), as further described below with reference to FIGURE 8. In a further embodiment, interstitial glucose testing and similar diagnostics supplement or replace manual self-testing, which provide a fully closed loop system when combined with a wireless insulin pump. Other management tool aspects are possible.

Automated Management of Type 2 Diabetes

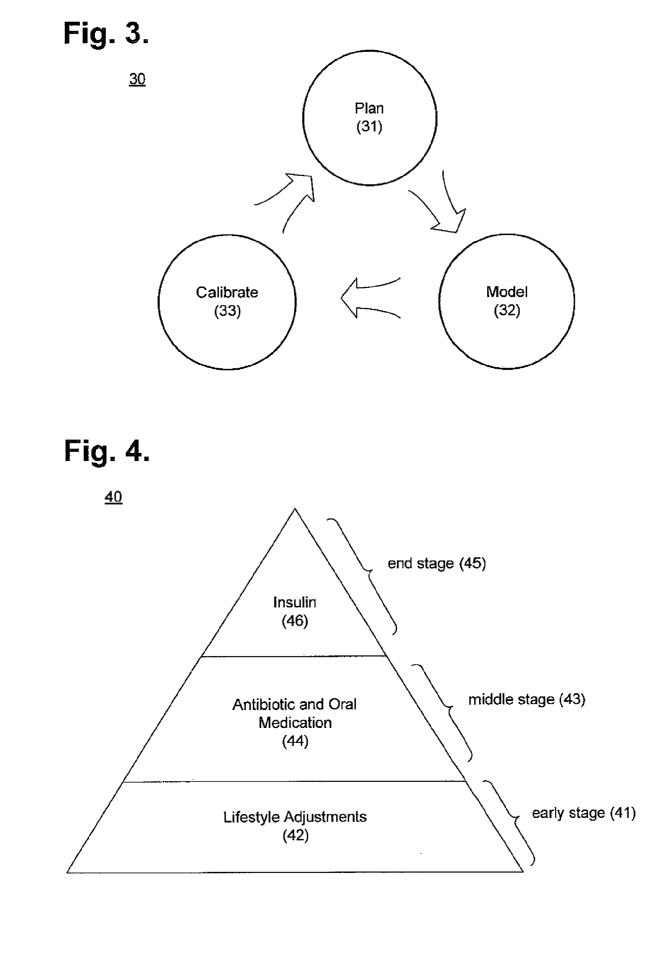

A diabetic patient is himself the best resource for managing his disease,. Meals, insulin dosing, antidiabetic and oral medication administration, and changes in personal well being, as well as departures from a regimen, are best known to the patient, who alone is ultimately responsible for compliance. FIGURE 3 is a process flow diagram showing personalized Type 2 diabetes mellitus modeling 30. The method is performed as a series of process steps or operations executed by one or more patient-operable devices, including personal computers, personal digital assistants, programmable mobile telephones, intelligent digital media players, or other programmable devices, that are working individually or collaboratively to apply a personal predictive management tool.

Modeling involves projecting the glycemic effects of planned meals and physical activities, which become increasingly important as the disease progresses. During the end-stage, the content and timing of meals greatly impacts blood glucose and is exclusively controlled by dosed insulin, which compensates for a lack of naturally-produced insulin. The management tool performs dietary planning (step 31), which involves determining the glycemic effect of food initially based on a standardized meal. In a further embodiment, planning also includes projecting the affect of exercise or physical activities that are likely to require appreciable caloric expenditure. Other planning aspects are possible.

Personalized models predict the timing and rise or fall of the patient's blood glucose in response to insulin, antidiabetic and oral medications, and food, as applicable. More particularly, once each planned meal is known, the management tool can model the time courses and amplitudes of blood glucose change for the meal (step 32). During the middle stage of the disease, the management tool further models antidiabetic and oral medications and, during the end-stage of the disease, dosed insulin. In a further embodiment, the management tool can be refined and calibrated as necessary based on empirical results and to adjust to self-testing recorded by the patient (step 33), such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference. Other modeling and calibrations are possible.

Progressive Management

Type 2 diabetes responds positively to strong management, which can be particularly effective during the early stage of the disease in checking or even reversing its progress.

FIGURE 4 is a diagram showing a method 40 for progressively managing the stages of Type 2 diabetes mellitus. The management tool follows the stages of the disease 41, 43, 45, and each stage-specific model builds on the prior stage.

One of the goals of Type 2 diabetes management is to provide close glycemic control, which directly influences long-term preservation of health and quality of life. Only lifestyle adjustments 42 are modeled during the early stage 41 and the management tool operates more as a personal "coach" than as a compliance monitor. Thereafter, antidiabetic and oral medications 44 are added to the model during the middle stage 43, as the patient's insulin resistance and production become impeded. Finally, the effects of dosed insulin 46 are modeled in the end-stage 45, which corresponds to a dependency on dosed insulin. Other stages of the management tool are possible, either in addition to or in lieu of the foregoing stages.

Early Stage

The treatment of early stage Type 2 diabetes centers on lifestyle adjustments, which helps to control blood glucose and lays the foundation for later stages of disease management. FIGURE 5 is a process flow diagram 60 showing management of the early stage of Type 2 diabetes mellitus for use with the method of FIGURE 4. In contrast to the middle and end-stages of management, the lifestyle adjustments are generally achieved without the introduction of antidiabetic or oral medications, or insulin therapy.

During early stage Type 2 diabetes, controlling blood glucose, blood pressure, and lipids are important. A patient is encouraged to exercise, watch his diet, and control his weight, as higher body fat increases insulin resistance and taxes the pancreas to produce more insulin to overcome the resistance caused by fat. Both food and exercise affect weight, and the management tool facilitates meal planning (step 51), which includes beverages, as an aid to weight management. A postprandial rise in blood glucose can be forecast (step 52) based on the patient's food selections, as further described below with reference to FIGURE 6. Generally, a Type 2 patient is urged to attain a postprandial blood glucose of 72-108 mg/dL with a two-hour postprandial blood glucose of 90-144 mg/dL. The amount and level of exercise or physical activity undertaken care can vary greatly between patients, and the management tool allows each patient to adjust the model for actual blood glucose (step 53) through application of empirical data as appropriate. For instance, a 12-mile bicycle ride might burn up 525 calories, which can be factored against the meals consumed during the course of the day as a counter to blood glucose rise and aid to lipid control. Other aspects of early stage management are possible.

Digestive Response Curve

The digestive response of each patient's body to food consumption is related to glycemic management, yet each patient is unique. A particular patient's digestive response characteristics can be normalized through consumption of a standardized test meal, such as a specific number of oat wafers, manufactured, for instance, by Ceapro Inc., Edmonton, Canada, or similar calibrated consumable. FIGURE 6 is a graph 60 showing, by way of example, a digestive response curve 61 for a standardized test meal. The x-axis represents time in minutes and the y-axis represents a cumulative rise of blood glucose measured in milligrams per deciliter (mg/dL). The amplitude of the curve 61 is patient-dependent, as is the timing of the rise. The test meal contains a known quantity of carbohydrate with a specific glycemic index. Thus, the curve 61 can be adapted to other types of foods to estimate glycemic effect and, during middle and end-stage Type 2 diabetes, counteraction of glycemic rise by antidiabetic and oral medication administration and insulin dosing. Other models of digestive response are possible.

Middle Stage

Although strong adherence to the lifestyle adjustments begun in early stage Type 2 diabetes can check or even reverse impaired insulin uptake, the body's predisposition to resist insulin usually returns at some later point in time. FIGURE 7 is a process flow diagram 70 showing management of the middle stage of Type 2 diabetes mellitus for use with the method of FIGURE 4. Middle stage Type 2 diabetes introduces antidiabetic and oral medications. As during early stage Type 2 management, the management tool facilitates meal planning (step 71) and additionally models any antidiabetic or oral medications taken (step 72). Most commonly, beguanide metformin, brand name Glucophage, and sulfonylureas, brand name Glucotrol, are prescribed to respectively help regulate inappropriate hepatic glucose release and stimulate insulin production. Thiazolidinediones, brand name Actos, may also be prescribed, which decreases insulin resistance. Other regimens of antidiabetic and oral medication are possible.

Meal planning and specifying antidiabetic and oral medications occur independently from meal consumption, as the timing and glycemic effect of the medications may only be indirectly related to postprandial blood glucose increase for specific meals. Thus, a postprandial rise in blood glucose is forecast (step 73) based only on the patient's food selections and not antidiabetic or oral medication effect. The model can further be adjusted for actual blood glucose (step 74) through application of empirical data as appropriate. Other aspects of middle stage management are possible, including the earlier "prophylactic" use of insulin in Type 2 diabetes, as some endocrinologists advise, before insulin therapy becomes required.

### End-stage

Insulin therapy is usually introduced during end-stage Type 2 diabetes, which compensates for the body's inability to naturally produce insulin. FIGURE 8 is a process flow diagram 80 showing management of the end-stage of Type 2 diabetes mellitus for use with the method of FIGURE 4. End-stage Type 2 diabetes management fundamentally centers on the timing and content of meals, and the timing and dosing of antidiabetic and oral medications, and insulin, although other factors, such as physical activity and exercise, patient well-being, illness, and stress, can influence the course of management.

As in the early and middle stages, meal planning (step 81) and any antidiabetic or oral medications taken are modeled (step 82). Additionally, with the assistance of the management tool, the patient determines a suitable dosage of insulin, which is dosed prior to the planned meal to counter the expected rise in blood glucose (step 83). A postprandial rise in blood glucose is forecast (step 84) and blood glucose self-testing results are entered (step 85) to refine and further calibrate the model. Other aspects of end-stage management are possible.

### Insulin Activity Curve

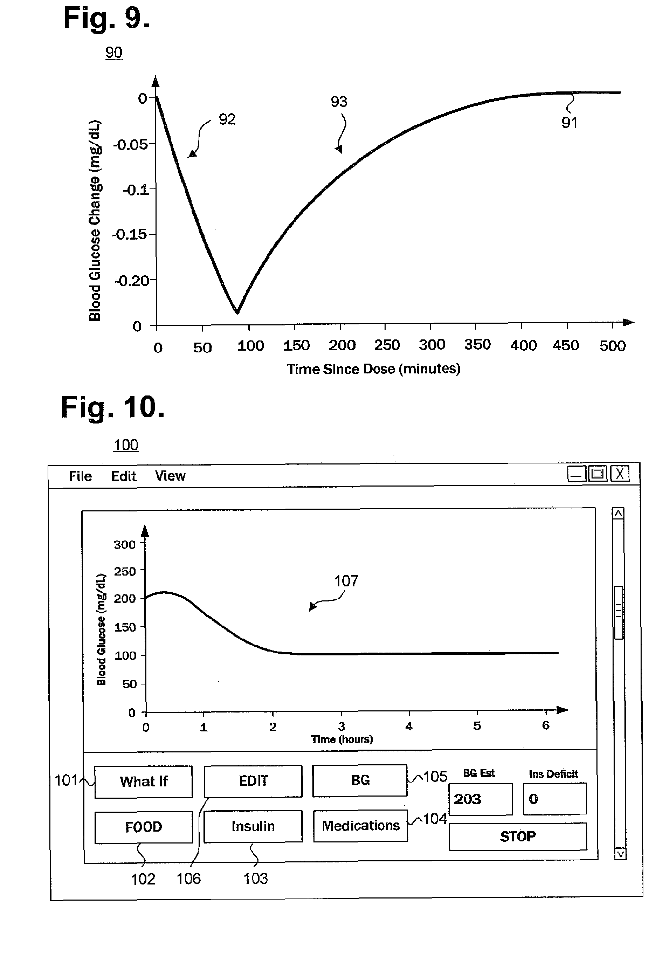

Like digestive response, insulin response is also dependent upon patient-specific sensitivities, which affect the time of onset, peak time, and duration of action of therapeutic effect. FIGURE 9 is a graph 90 showing, by way of example, a personal insulin activity curve. The x-axis represents time in minutes and the y-axis represents incremental blood glucose decrease measured in mg/dL. The personal insulin activity model can be depicted through an approximation of population-based insulin activity curves published by insulin manufacturers and other authoritative sources. The patient-specific insulin activity curve 91 mimics the shape of the population-based insulin activity curves through a curvilinear ramp 92 formed to peak activity time, followed by an exponential decay $\tau$ 93. Thus, for a patient insulin sensitivity coefficient of 90%, a patient-specific insulin activity curve 91 would reflect a ten percent decrease in insulin sensitivity over corresponding population-based insulin activity curve results. Other models of insulin activity are possible.

### Graphical User Interface

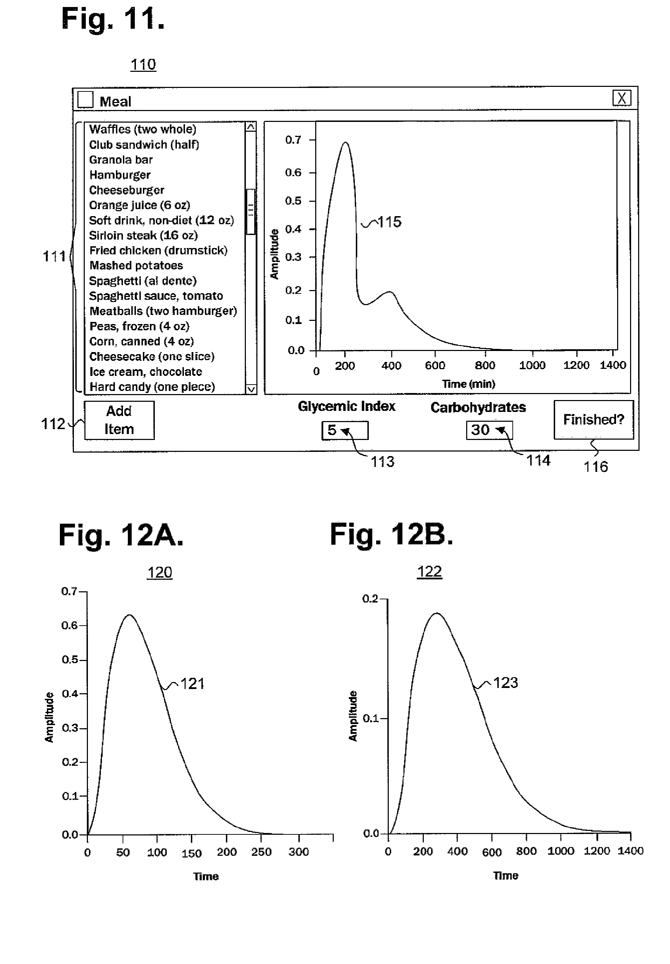

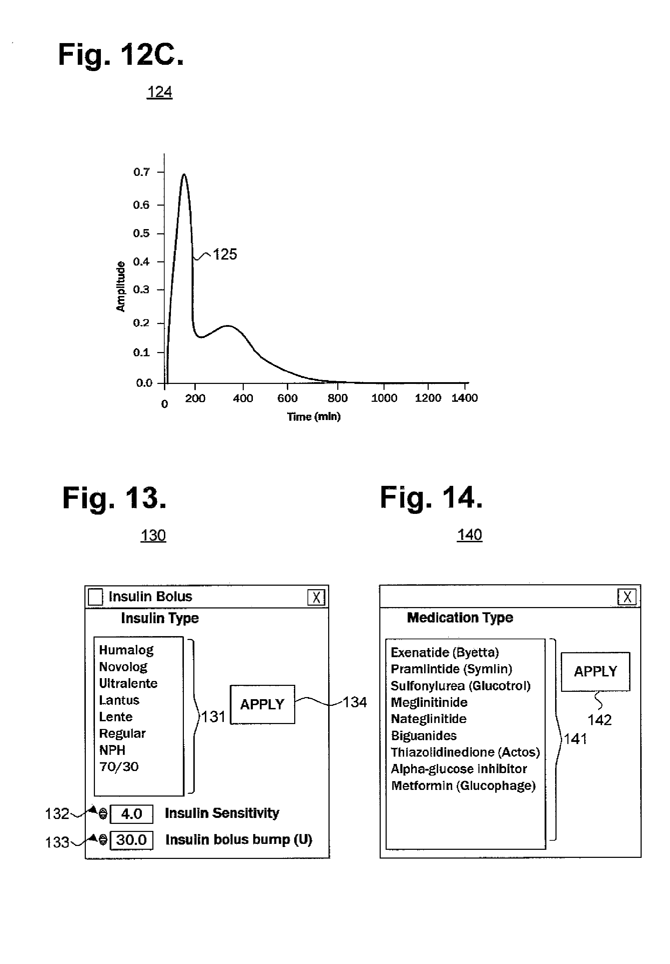

Personalized Type 2 diabetes mellitus management can be provided through a patient-operable interface through which glycemic effect prediction and patient interaction can be performed. FIGURE 10 is a diagram showing, by way of example, a screen shot of a graphical user interface 100 for performing automated management of Type 2 diabetes, in accordance with one embodiment. The user interface 100 provides logical controls that accept patient inputs and display elements that present information to the patient. The logical controls include buttons, or other forms of option selection, to access further screens and menus to estimate glucose rise and insulin needed to counteract the rise 101 ("What IP'); plan meals 102 ("FOOD"), as further described below with reference to FIGURE 11; specify an insulin bolus 103 ("Insulin"), as further described below with reference to FIGURE 13; specify other antidiabetic and oral medications 104 ("Medications"), as further described below with reference to FIGURE 14; enter a measured blood glucose reading 105 ("BG"); and edit information 106 ("EDIT"). Further logical control and display elements are possible.

To assist the patient with planning, a graphical display provides a forecast curve 107, which predicts combined insulin dosing, antidiabetic and oral medication administration, and postprandial blood glucose, as applicable, depending on the disease stage. The x-axis represents time in hours and the y-axis represents the blood glucose level measured in mg/dL. Modeling estimates the timing and amplitude of change in the patient's blood glucose in response to the introduction of a substance, whether food, physiological state, or drug, that triggers a physiological effect in blood glucose. Generally, actions, such as insulin dosing, medication administration, exercise, and food consumption cause a measureable physiological effect, although other substances and events can influence blood glucose. The time courses and amplitudes of change are adjusted, as appropriate, to compensate for patient-specific factors, such as level of sensitivity or resistance to insulin, insulin secretion impairment, carbohydrate sensitivity, and physiological reaction to medications. In a further embodiment, the management tool includes a forecaster that can identify a point at which an expected blood glucose level from the personal insulin response profile is expected to either exceed or fall below a blood glucose level threshold, which respectively corresponds to hypoglycemia and hyperglycemia. Other actions and patient-specific factors, like exercise or supervening illness, may also alter the time courses and amplitudes of blood glucose.

In one embodiment, a meal is planned through a food selection user interface, as further described below with reference to FIGURE 11, and insulin dosing is estimated through an insulin selection user interface, as further described below with reference to FIGURE 13. The digestive response, insulin, and any other medication activity curves are combined, so the effect of the insulin dosing and other drugs, if applicable, can be weighed against the proposed meal. Other forecasting aids are possible.

In one embodiment, the user interface 100 and related components are implemented using a data flow programming language provided through the LabVIEW development platform and environment, licensed by National Instruments Corporation, Austin, TX although other types and forms of programming languages, including functional languages, could be employed. The specific option menus will now be discussed.

Food Selection

Estimating postprandial glucose rise involves modeling food constituents as combined into a meal of specific food types, portion sizes, and preparations. FIGURE 11 is a diagram showing, by way of example, a screen shot of a graphical user interface 110 for selecting food combinations for use in the graphical user interface 100 of FIGURE 10. Dietary management, and thence, the management tool, focuses on carbohydrates, which have the greatest affect on blood glucose. Simple sugars increase blood glucose rapidly, while complex carbohydrates, such as whole grain bread, increase blood glucose more slowly due to the time necessary to break down constituent components. Fats, whether triglyceride or cholesterol, neither raise nor lower blood glucose, but can have an indirect effect by delaying glucose uptake. Proteins are broken down into amino acids, which are then converted into glucose that will raise blood glucose levels slowly. Proteins will not generally cause a rapid blood glucose rise. Nevertheless, both fats and proteins are incorporated into the model by virtue of their empiric effect on blood glucose levels. Additionally, various combinations or preparations of medications or food can have synergistic effects that can alter blood glucose rise and timing.

In the management tool, different meal combinations can be composed by selecting individual foods from a food data library, which stores glycemic effect, digestive speeds and amplitudes as a function of carbohydrate content. The food data library is displayed as food choices 111. For convenience, portion size and preparation, where applicable, are included with each food choice 111, although portion size and preparation could alternatively be separately specified.

The food choices 111 are open-ended, and one or more food items can be added to a planned meal by pressing the "ADD ITEM" button 112. Glycemic effect data, such as the glycemic index 113 and carbohydrate type and content 114 for a particular food item, are retrieved also from the stored food data library and displayed. A cumulative digestive response curve 115 is generated, as further described below with reference to FIGURES 12A-C. The digestive response curve 115 estimates digestive speed and amplitude for the individual patient, which traces postprandial blood glucose rise, peak, and fall based on the patient's carbohydrate sensitivity. The carbohydrate sensitivity can be expressed as a coefficient or other metric that can be applied to population-based glycemic effect data, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference. The completion of meal planning is indicated by pressing the "Finished" button 116. Further logical control and display elements are possible.

Constituent Digestive Response

A planned meal must be evaluated to determine the insulin needed to compensate for the estimated postprandial rise in blood glucose. FIGURES 12A-C are graphs 120,122, 124 showing, by way of example, constituent and cumulative digestive response curves 121, 123, 125 for a hypothetical meal. The x-axes represent time in minutes and the y-axes represent cumulative rise of blood glucose measured in milligrams per deciliter (mg/dL). The amplitude of the curves 121, 123, 125 are patient-dependent, as is the timing of the rise.

In general, food consumption modeling focuses on carbohydrates. Simple sugars, the most basic form of carbohydrate, increase blood glucose rapidly. Conversely, more complex carbohydrates, such as whole grain bread, increase blood glucose more slowly due to the time necessary to break down constituent components. Proteins also raise blood glucose slowly, as they must first be broken down into amino acids before being converted into glucose. Fats, which include triglycerides and cholesterol, delay glucose uptake. Thus, carbohydrates, and not proteins or fats, have the largest and most direct affect on blood glucose. Notwithstanding the relative glycemic index of a type of food, all foods that contribute to blood glucose rise, not just carbohydrates, can be included in the model.

Each item of food consumed contributes to the overall carbohydrate content and, thence, postprandial blood glucose rise. Referring first to FIGURE 12A, a graph 120 showing, by way of example, a digestive response curve 121 for postprandial blood glucose rise for a six ounce glass of orange juice is provided. The curve 121 reflects a relatively fast and pronounced rise in blood glucose, which peaks about an hour following consumption. Referring next to FIGURE 12B, a graph 122 showing, by way of example, a digestive response curve 123 for postprandial blood glucose rise for a 16 ounce sirloin steak is provided. The curve 123 reflects a comparatively prolonged and modest rise in blood glucose, which peaks about five-and-a-half hours following consumption.

The type of food and manner of preparation can affect glucose uptake. Orange juice is a beverage that is readily metabolized and absorbed into the blood stream, which results in a rapid and significant rise in blood glucose. The rise, though, is short term. In contrast, steak is primarily protein and the manner of preparation will have little effect on carbohydrate content. The rise in blood glucose is delayed by the protein having to first be broken down into amino acids. The resultant equivalent carbohydrate content also is low, thus resulting in a more attenuated rise in blood glucose. Food items principally containing complex carbohydrates are more affected by manner of preparation. For example, pasta prepared "al dente" is slightly undercooked to render the pasta firm, yet not hard, to the bite. The "al dente" form of preparation can increase digestive time and delay glucose uptake. The form of preparation can also be taken into account in the management tool. Finally, some medications can modify the effect of foods on blood glucose. Other effects on food items, as to type and manner of preparation, also are possible.

Cumulative Digestive Response

Except for the occasional snack item, food is generally consumed as a meal. Items of food consumed in combination during a single sitting, as typical in a meal, can cumulatively or synergistically interact to alter the timing and amplitude of blood glucose rise based on the digestive processes involved and the net change to overall carbohydrate content. Referring finally to FIGURE 12C, a graph 124 showing, by way of example, a cumulative digestive response curve 125 for postprandial blood glucose rise for a meal that combines the six ounce glass of orange juice with the 16 ounce sirloin steak is provided. The cumulative digestive response curve 125 combines the respective constituent digestive response curves 121, 123 and proportionately applies the patient's carbohydrate sensitivity. The cumulative curve has an initial near-term peak, which reflects the short time course and high glucose content of the orange juice, and a delayed long term peak, which reflects the protein-delayed and significantly less-dramatic rise in blood glucose attributable to the sirloin steak. Shortly following consumption of the orange juice and steak, blood glucose rise is dominated by the effects of the orange juice while the steak has little effect. Later, the effects of the orange juice dwindle and the effects of the steak dominate the rise in blood glucose. The effects of both foods are present in-between.

The cumulative digestive $\vec{r}$ response can be determined by taking a summation of the constituent digestive responses over the estimated time course adjusted for synergistic effect:

$$\vec{r} = \sum_{i=1}^{n} \vec{d}_i k \qquad (1)$$

where $d_i = \{x_1, x_2, ..., x_m\}$, such that there are n constituent digestive response vectors, each normalized to length m, and containing digestive response values x; and k is an adjustment coefficient for synergy, such that $k > 0$. The last element of each constituent digestive response vector is repeated to ensure all constituent digestive response vectors are of the same length. Other cumulative digestive response determinations are possible.

The particular combinations of orange juice and steak have little synergistic effect. The orange juice, as a beverage, metabolizes quickly in the stomach, whereas the steak, as a solid protein, is primarily metabolized in the small intestine following secretion of bile. Other food combinations, though, can synergistically raise or lower the overall carbohydrate level, or accelerate or delay glucose uptake.

Insulin Selection

The selections of insulin and other medications, when applicable, are also key to diabetes management. When under a dosed insulin regimen, the patient needs to identify both the type and amount of insulin preparation used and his sensitivity to allow the management tool to generate an insulin response curve. Insulin preparation types are identified by source, formulation, concentration, and brand name, and are generally grouped based on duration of action. FIGURE 13 is a diagram showing, by way of example, a screen shot of a graphical user interface 130 for specifying insulin preparation type for use in the graphical user interface 100 of FIGURE 10. Different types of insulin preparation 131 can be selected and, for ease of use and convenience, are identified by brand name or formulation. Insulin preparations include short-acting insulins, such as lispro or Regular, are used to cover a meal and are frequently administered by insulin pump due to the short time of onset. Intermediate-acting insulins, such as NPH (Neutral Protamine Hagedorn), have 12-18 hour durations, which peak at six to eight hours. Finally, long-acting insulins, such as Ultralente, have 32-36 hour durations to provide a steady flow of insulin. Long-acting insulins are generally supplemented with short-acting insulins taken just before meals. Other types of insulin preparations include insulin glargine, insulin detemir, and insulin preparation mixes, such as "70/30," which is a premixed insulin preparation containing 70% NPH insulin and 30% Regular insulin. In addition, insulin sensitivity 132 and insulin bolus "bump" 133, that is, a single dosing, such as for meal coverage, can be specified, before being factored into the model upon pressing of the "APPLY" button 134. The insulin response curve is adjusted based on the patient's insulin sensitivity. The insulin sensitivity can be expressed as a coefficient or other metric that can be applied to published insulin activity curves, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. _____, pending, the disclosures of which are incorporated by reference. Further logical control and display elements are possible.

Other Medication Selection

Type 2 diabetics often receive antidiabetic and oral medications during the middle and end-stages of the disease. Each such medication should also be identified to allow the management tool to project any effect on glycemic activity. A patient may currently be taking medications in addition to insulin. FIGURE 14 is a diagram showing, by way of example, a screen shot of a graphical user interface 140 for specifying other medications for use in the graphical user interface 100 of FIGURE 10. Different medications 141 can be selected and, for ease of use and convenience, can be identified by generic name, brand name, or formulation. As appropriate, the therapeutic effects, particularly as relating to blood glucose level and drug interactions of each medication can be factored into the model upon pressing of the "APPLY" button 142. For example, pramlintide acetate, offered under the brand name Symlin, is prescribed to both Type 1 and Type 2 diabetics help lower postprandial blood glucose during the three hours following a meal. Consequently, the blood glucose rise for a Type 1 diabetic would need to be adjusted to reflect the effects of the pramlintide acetate in light of a planned meal and dosed insulin. Further logical control and display elements are possible.

Method

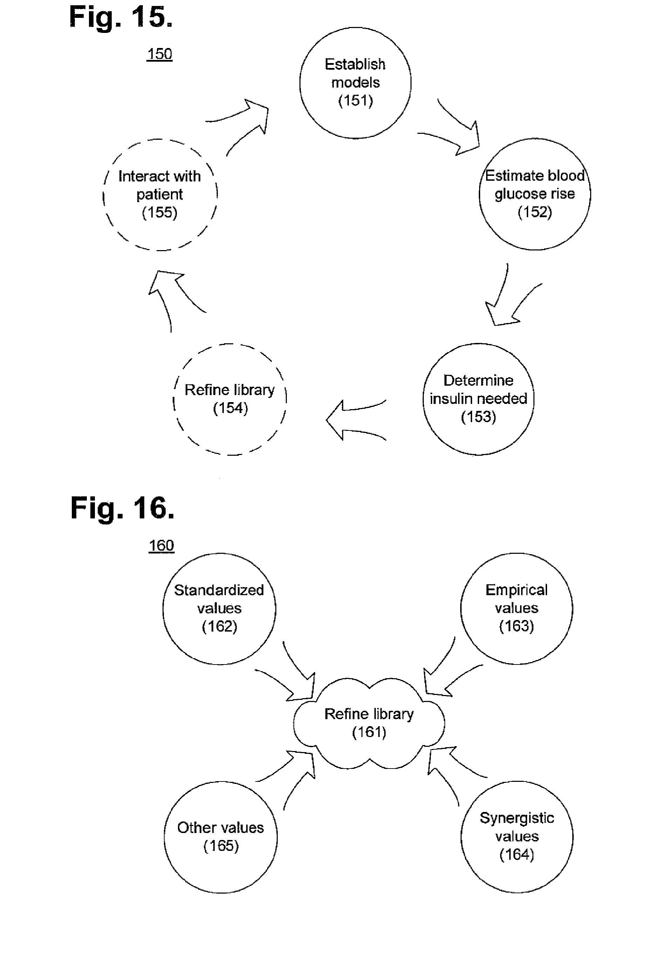

Conventional Type 2 diabetes management relies on patient intuition and experiential awareness of antidiabetic medication and insulin sensitivities. Individualized diabetes management can be significantly improved by modeling quantified patient food and drug sensitivities. FIGURE 15 is a process flow diagram showing a method for actively managing Type 2 diabetes mellitus on a personalized basis 150, in accordance with one embodiment. Active management proceeds as a cycle of repeated operations that are reflective of basic day-to-day diabetes control. Initially, a personal predictive management tool is established (operation 151), which models food, antidiabetic and oral medication, and insulin sensitivities, such as described in commonly-assigned U.S. Patent application, entitled "System And Method For Creating A Personalized Tool Predicting A Time Course Of Blood Glucose Affect In Diabetes Mellitus," Serial No. _____, pending, and U.S. Patent application, entitled "System And Method For Generating A Personalized Diabetes Management Tool For Diabetes Mellitus," Serial No. , pending, the disclosures of which are incorporated by reference. Thereafter, a rise in blood glucose is estimated (operation 152) by determining a cumulative digestive response curve based on the patient's food selections, as described above with reference to FIGURES 12A-C. During end-stage Type 2 diabetes, the insulin dosage needed to counteract the rise in blood glucose is determined (operation 153) based on the cumulative digestive response curve. The dosage can be estimated, for instance, through a graphical display that provides a forecast curve 107 (shown in FIGURE 10), which predicts combined insulin dosing and postprandial blood glucose. Other insulin dosing estimates are possible.

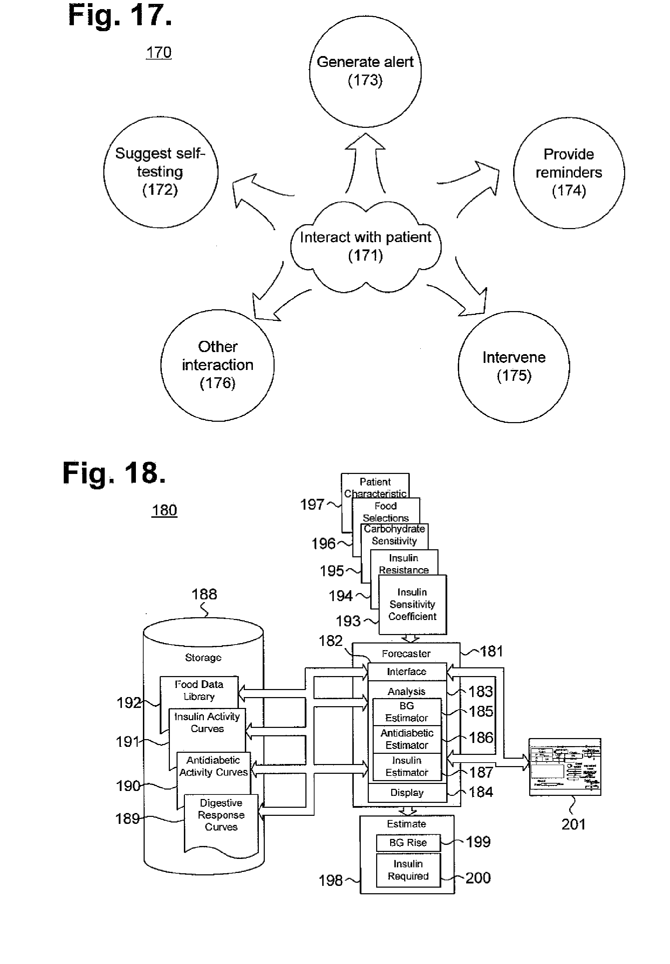

In a further embodiment, the food data library can be refined to add new food items or to revise the food data (operation 154), as further described below respectively with reference to FIGURE 16. In a still further embodiment, the management tool can directly interact with the patient (operation 155), as further described below respectively with reference to FIGURE 17. The active management operations can be repeated as needed.

Food Data Library Refinement

Both the types of available food items and their accompanying data may change over time. FIGURE 16 is a process flow diagram showing a routine for refining a food data library 160 for use with the method 150 of FIGURE 15. At a minimum, the food data library 161 contains glycemic effect, digestive speeds and amplitudes as a function of carbohydrate content. The data can be obtained from various sources and is integrated into the library 161. For instance, standardized carbohydrate values 162, for instance, glycemic indices or glycemic load, can be retrieved from authoritative sources, such as the University of Toronto, Toronto, Ontario, Canada. Empirical values 163 can be derived by the patient through experiential observations of glycemic effect by a combination of fasting and pre- and postprandial blood glucose testing. Synergistic values 164 of food combinations, perhaps unique to the patient's personal culinary tastes, could similarly be empirically derived. Other food data values 165 and sources of information are possible.

Patient Interaction

In the course of providing blood glucose management, a more proactive approach can be taken as circumstances provide. FIGURE 17 is a process flow diagram showing a routine for interacting with a patient 170 for use with the method 150 of FIGURE 15. Interaction refers to the undertaking of some action directly with or on behalf of the patient. The interaction can include suggesting opportune times to the patient at which to perform self-testing of blood glucose (operation 172). Such times include both pre- and postprandial times, particularly when blood glucose rise is estimated to peak. Similarly, alerts can be generated (operation 173), for example, warnings of low blood glucose, or reminders provided (operation 174), such as reminding the patient to take his antidiabetic or oral medication or insulin for high glucose levels, if applicable. Interaction could also include intervening (operation 175), such as notifying a patient's physician or emergency response personnel when a medical emergency arises. Other forms of patient interaction (176) are possible.

System

Automated Type 2 diabetes management can be provided on a system implemented through a patient-operable device, as described above with reference to FIGURE 3. FIGURE 18 is a block diagram showing for a system for actively managing Type 2 diabetes mellitus on a personalized basis 180, in accordance with one embodiment. At a minimum, the patient-operable device must accommodate user inputs, provide a display capability, and include local storage and external interfacing means.

In one embodiment, the system 180 is implemented as a forecaster application 181 that includes interface 182, analysis 183, and display 184 modules, plus a storage device 188. Other modules and devices are possible.

The interface module 182 accepts user inputs, such as insulin sensitivity coefficients 193, insulin resistance 194, carbohydrate sensitivity 195, food selections 196, and patient-specific characteristics 197. Other inputs, both user-originated and from other sources, are possible. In addition, in a further embodiment, the interface module 182 facilitates direct interconnection with external devices, such as a blood or interstitial glucose monitor, or centralized server (not shown). The interface module 182 can also provide wired or wireless access for communication over a private or public data network, such as the Internet. Other types of interface functionality are possible.

The analysis module 183 includes blood glucose estimator 185, antidiabetic estimator 186, and insulin estimator 187 submodules. The blood glucose estimator submodule 185 forms a personal digestive response curve 188, which is determined from data in the food data library 191 for the food selections 196. The personal digestive response curve 188 can be determined using glycemic effect, digestive speeds and amplitudes as a function of the carbohydrate sensitivity 195. The antidiabetic estimator 186 forms an antidiabetic and oral medications activity curve 190 based on drug profile and insulin resistance 194. Similarly, the insulin estimator 187 forms an insulin activity curve 191 using, for instance, a population-based insulin activity curve proportionately adjusted by the insulin sensitivity coefficient 193. The personal digestive response curve 189, antidiabetic and oral medication activity curve 190, and insulin activity curve 191 are used by the analysis module 183 to generate an estimate 198 of blood glucose rise 199 and insulin required 200, as applicable. Other analytical functions are possible.

Finally, the display module 184 generates a graphical user interface 201, through which the user can interact with the forecaster 181. Suggestions for blood glucose self-testing times, alerts, and reminders are provided via the display module 184, which can also generate an intervention on behalf of the patient. The user interface 201 and its functionality are described above with reference to FIGURE 10.

While the invention has been particularly shown and described as referenced to the embodiments thereof, those skilled in the art will understand that the foregoing and other changes in form and detail may be made therein without departing from the spirit and scope.

**Claims**

**1.** A system (180) for actively managing Type 2 diabetes mellitus on a personalized basis, comprising:

a database (188) comprising a model of glycemic effect for a Type 2 diabetic patient (31) for digestive response (61); and

a forecaster module (181), comprising:

an adjustment module (183) configured to adjust the digestive response model (61) for a degree of insulin resistance (194) experienced by the patient (31); and

a blood glucose estimator module (185) configured to estimate a rise in postprandial blood glucose (121) through food ingestion of a planned meal (102) in proportion to the adjusted digestive response model (61).

2. A system (180) according to Claim 1, further comprising:

a model of glycemic effect for the patient (31) for physical activity further comprised in the database (188), wherein the digestive response model (61) is adjusted for a degree of insulin resistance (194) experienced by the patient (31) by factoring in the physical activity model.

3. A system (180) according to Claim 1, further comprising:

a model of glycemic effect for the patient (31) for a time course of an antidiabetic medication further comprised in the database (188), wherein an amount of the antidiabetic medication necessary to counter the degree of insulin resistance (194) is determined by applying the antidiabetic medication model against the adjusted digestive response model (61).

4. A system (180) according to Claim 1, further comprising:

a model of glycemic effect for the patient (31) for a time course of insulin further comprised in the database (188), wherein an amount of insulin necessary and timing of delivery of that insulin to mediate transport of blood glucose into cells in proportion to the postprandial blood glucose rise is determined through the insulin time course model.

5. A system (180) according to Claim 1, further comprising:

a library of digestive responses for foods (189), which include rises in blood glucose particular to the patient (31), wherein the digestive responses for the foods in the planned meal (102) are aggregated over overlapping time courses as the digestive response model (61).

6. A system (180) according to Claim 5, wherein the library (189) is maintained as glycemic indices, and the glycemic indices for the foods in the planned meal (102) are apportioned as glycemic loads based on portion size.

7. A system (180) according to Claim 5, further comprising:

a determination module configured to determine the digestive response model (61) as a summation of the digestive responses for the foods in the planned meal (102).

8. A method for actively managing Type 2 diabetes mellitus on a personalized basis, comprising:

establishing a model of glycemic effect for a Type 2 diabetic patient (31) for digestive response (61);
adjusting the digestive response model (61) for a degree of insulin resistance (194) experienced by the patient (31); and
estimating a rise in postprandial blood glucose (121) through food ingestion of a planned meal (102) in proportion to the adjusted digestive response model (61).

9. A method according to Claim 8, further comprising:

establishing a model of glycemic effect for the patient (31) for physical activity; and
adjusting the digestive response model (61) for a degree of insulin resistance (194) experienced by the patient (31) by factoring in the physical activity model.

10. A method according to Claim 8, further comprising:

establishing a model of glycemic effect for the patient (31) for a time course of an antidiabetic medication; and determining an amount of the antidiabetic medication necessary to counter the degree of insulin resistance (194) by applying the antidiabetic medication model against the adjusted digestive response model (61).

11. A method according to Claim 8, further comprising:

establishing a model of glycemic effect for the patient (31) for a time course of insulin; and determining an amount of insulin necessary and timing of delivery of that insulin to mediate transport of blood glucose into cells in proportion to the postprandial blood glucose rise through the insulin time course model.

12. A method according to Claim 8, further comprising:

referencing a library of digestive responses for foods, which include rises in blood glucose particular to the patient (31); and aggregating the digestive responses for the foods in the planned meal (102) over overlapping time courses as the digestive response model (61).

13. A method according to Claim 12, further comprising:

maintaining the library as glycemic indices; and apportioning the glycemic indices for the foods in the planned meal (102) as glycemic loads based on portion size.

14. A method according to Claim 12, further comprising:

determining the digestive response model (61) as a summation of the digestive responses for the foods in the planned meal (102).

Fig. 1B. (prior art)

16

Fig. 1A. (Prior Art)

10

# Fig. 1C (prior art).

19

# Fig. 2A.

30

EP 2 354 991 A1

# Fig. 2B.

37

31

38

39

40

# Fig. 2C.

41

31 42

44

43

47

45

46

EP 2 354 991 A1

16

# Fig. 3.

30

Plan
(31)

Calibrate
(33)

Model
(32)

# Fig. 4.

40

end stage (45)

Insulin
(46)

middle stage (43)

Antibiotic and Oral
Medication
(44)

early stage (41)

Lifestyle Adjustments
(42)

# Fig. 5.

50

# Fig. 6.

60

# Fig. 7.

70

Plan meals (71)

Forecast BG change (73)

Take antidiabetic and oral medications (72)

Empirically adjust BG (74)

# Fig. 8.

80

Plan meals (81)

Dose insulin (83)

Take antidiabetic and oral medication (82)

Forecast BG change (84)

Self-test BG (85)

# Fig. 9.

90

# Fig. 10.

100

# Fig. 11.

110

| Meal | | X |

111
- Waffles (two whole)
- Club sandwich (half)
- Granola bar
- Hamburger
- Cheeseburger
- Orange juice (6 oz)
- Soft drink, non-diet (12 oz)
- Sirloin steak (16 oz)
- Fried chicken (drumstick)
- Mashed potatoes
- Spaghetti (al dente)
- Spaghetti sauce, tomato
- Meatballs (two hamburger)
- Peas, frozen (4 oz)
- Corn, canned (4 oz)
- Cheesecake (one slice)
- Ice cream, chocolate
- Hard candy (one piece)

~115

112 Add Item

Glycemic Index    Carbohydrates

5    30    Finished?

113        114        116

# Fig. 12A.

120

~121

# Fig. 12B.

122

~123

# Fig. 12C.

124

125

# Fig. 13.

130

Insulin Bolus [X]

## Insulin Type

Humalog
Novolog
Ultralente
Lantus       APPLY       134
Lente     131
Regular
NPH
70/30

132  ▲⊝ 4.0  Insulin Sensitivity
133  ▲⊝ 30.0  Insulin bolus bump (U)

# Fig. 14.

140

Medication Type [X]

Exenatide (Byetta)
Pramlintide (Symlin)               APPLY
Sulfonylurea (Glucotrol)
Meglinitinide                        142
Nateglinitide
Biguanides                    141
Thiazolidinedione (Actos)
Alpha-glucose inhibitor
Metformin (Glucophage)

# Fig. 15.

150

Establish
models
(151)

Estimate blood
glucose rise
(152)

Interact with
patient
(155)

Determine
insulin needed
(153)

Refine library
(154)

# Fig. 16.

160

Standardized
values
(162)

Empirical
values
(163)

Refine library
(161)

Other values
(165)

Synergistic
values
(164)

# Fig. 17.

170

Generate alert
(173)

Suggest self-
testing
(172)

Provide
reminders
(174)

Interact with patient
(171)

Other
interaction
(176)

Intervene
(175)

# Fig. 18.

180

Patient
Characteristic

197

Food
Selections

196

Carbohydrate
Sensitivity

195

Insulin
Resistance

194

Insulin
Sensitivity
Coefficient

193

188

Storage

182

Forecaster ~181

Interface

Analysis ~183

Food Data
Library

192

BG
Estimator ~185

Insulin Activity
Curves

191

Antidiabetic
Estimator ~186

Antidiabetic
Activity Curves

190

Insulin
Estimator ~187

Digestive
Response
Curves

189

Display ~184

201

Estimate

BG Rise ~199

Insulin
Required ~200

198

Europäisches
Patentamt

European
Patent Office

Office européen
des brevets

# EUROPEAN SEARCH REPORT

Application Number

EP 10 15 2575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/208113 A1 (MAULT JAMES R [US] ET AL) 6 November 2003 (2003-11-06)<br>* paragraph [0014] - paragraph [0015] *<br>* paragraph [0021] - paragraph [0023] *<br>* paragraph [0039] *<br>* paragraph [0043] *<br>* paragraph [0076] *<br>* paragraph [0125] - paragraph [0128] *<br>----- | 1-14 | INV.<br>G06F19/00 |
| X | LEHMANN E D ET AL: "AN INTEGRATED APPROACH FOR THE COMPUTER-ASSISTED TREATMENT OF DIABETES PATIENTS ON INSULIN",<br>MEDICAL INFORMATICA, TAYLOR AND FRANCIS, BASINGSTOKE, GB,<br>vol. 17, no. 2, 1 April 1992 (1992-04-01), pages 105-123, XP002052427,<br>ISSN: 0307-7640<br>* the whole document *<br>----- | 1-14 | |
| X | SALZSIEDER E ET AL: "A model-based system for the individual prediction of metabolic responses toimprove the therapy in type I diabetes",<br>HORMONE AND METABOLIC RESEARCH, THIEME-STRATTON, STUTTGART, DE,<br>vol. 24, no. SUPPL,<br>1 January 1990 (1990-01-01), pages 10-19, XP008090314,<br>ISSN: 0018-5043<br>* the whole document *<br>----- | 1-14 | TECHNICAL FIELDS SEARCHED (IPC)<br><br>G06F |
| X | EP 0 834 825 B1 (SALZSIEDER ECKHARD DR [DE]; RUTSCHER ALEXANDER DIPL ING [DE] SALZSIEDE) 14 April 2004 (2004-04-14)<br>* claim 1 *<br>* paragraph [0033] *<br>----- | 1-14 | |

-/--

The present search report has been drawn up for all claims

| Place of search | Date of completion of the search | Examiner |
|---|---|---|
| Munich | 19 November 2010 | Sisk, Aisling |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 10 15 2575 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 822 715 A (WORTHINGTON DAVID R L [US] ET AL) 13 October 1998 (1998-10-13) * claim 1 *<br>----- | 1-14 | |
| X | EP 2 023 256 A1 (NOVO NORDISK AS [DK]) 11 February 2009 (2009-02-11) * paragraph [0020] - paragraph [0021] * * paragraph [0036] * * paragraph [0060] *<br>----- | 1-14 | |
| | | | TECHNICAL FIELDS SEARCHED (IPC) |

The present search report has been drawn up for all claims

| Place of search | Date of completion of the search | Examiner |
|---|---|---|
| Munich | 19 November 2010 | Sisk, Aisling |

EPO FORM 1503 03.82 (P04C01)

**ANNEX TO THE EUROPEAN SEARCH REPORT
ON EUROPEAN PATENT APPLICATION NO.**

EP 10 15 2575

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2010

| Patent document cited in search report | | Publication date | Patent family member(s) | | Publication date |
|---|---|---|---|---|---|
| US 2003208113 | A1 | 06-11-2003 | NONE | | |
| EP 0834825 | B1 | 14-04-2004 | DE | 19634577 A1 | 05-03-1998 |
| | | | EP | 0834825 A1 | 08-04-1998 |
| US 5822715 | A | 13-10-1998 | AU | 1309700 A | 17-04-2000 |
| | | | WO | 0018293 A1 | 06-04-2000 |
| | | | US | 6379301 B1 | 30-04-2002 |
| | | | US | 6167362 A | 26-12-2000 |
| | | | US | 6233539 B1 | 15-05-2001 |
| | | | US | 5956501 A | 21-09-1999 |
| EP 2023256 | A1 | 11-02-2009 | CA | 2693122 A1 | 05-02-2009 |
| | | | CN | 101772769 A | 07-07-2010 |
| | | | EP | 2186032 A1 | 19-05-2010 |
| | | | WO | 2009016050 A1 | 05-02-2009 |
| | | | US | 2010280329 A1 | 04-11-2010 |

EPO FORM P0459

For more details about this annex : see Official Journal of the European Patent Office, No. 12/82

**EP 2 354 991 A1**

**REFERENCES CITED IN THE DESCRIPTION**

**Patent documents cited in the description**

- US 6168563 B, Brown
- US 6024699 A, Surwit